# EUROPÄISCHE PATENTSCHRIFT

(11) **EP 0 497 781 B1**
(45) Veröffentlichungstag und Bekanntmachung des Hinweises auf die Patenterteilung: **12.01.1994**
(21) Anmeldenummer: 90913154.2
(22) Anmeldetag: 11.09.1990
(51) Int. Cl.: A61B 1/30, A61M 25/01, A61B 1/12

(54) **EINFÜHRVORRICHTUNG FÜR SCHLAUCHFÖRMIGE FIBEROPTISCHE INSTRUMENTE, INSBES. KOLONOSKOPE**
DEVICE FOR INSERTING TUBULAR OPTICAL FIBRE INSTRUMENTS, ESPECIALLY COLONOSCOPES
DISPOSITIF D'INTRODUCTION POUR APPAREILS TUBULAIRES EN FIBRES OPTIQUES, NOTAMMENT POUR COLONOSCOPE

(30) Priorität: 23.10.1989 DE 3935256
(43) Veröffentlichungstag der Anmeldung: 12.08.1992
(73) Patentinhaber: BAUERFEIND, Peter, D-84478 Waldkraiburg (DE)
(72) Erfinder: BAUERFEIND, Peter, D-8264 Waldkraiburg (DE); BAUERFEIND, Herbert, D-8264 Waldkraiburg (DE)
(74) Vertreter: Goetz, Rupert, Dipl.-Ing.
(86) Internationale Anmeldenummer: EP9001532
(87) Internationale Veröffentlichungsnummer: WO9105507

(56) Entgegenhaltungen:
- EP-A- 371 486
- DE-A- 3 704 247
- DE-A- 3 900 738
- US-A- 4 815 450

## Beschreibung

Die Erfindung betrifft eine Einführvorrichtung für schlauchförmige fiberoptische Instrumente, insbes. Kolonoskope, Gastroskope und dgl. mit einem Griffteil und einem in ein Untersuchungsobjekt, insbes. menschliches Kolon, einschiebbaren, biegsamen Einschubteil, der zwischen einer Innenwand und einer Außenwand mindestens einen nach außen abgedichteten Zwischenraum aufweist, in den sich ein Fluid einleiten läßt, wobei
- die Außenwand des Einschubteils von einem flexiblen, jedoch vom Fluid im Zwischenraum nicht ballonartig aufblähbaren Schlauch gebildet ist,
- die Innenwand des Einschubteils ebenfalls von einem Schlauch gebildet ist, und
- der Zwischenraum Stützkörper enthält, über die sich die Innenwand und die Außenwand aneinander abstützen, wenn der Druck im Zwischenraum einen vorbestimmten Betrag unterschreitet.

Eine Einführvorrichtung dieser Gattung ist aus der US 4815450 bekannt. Dort enthält der Zwischenraum zwischen Innen- und Außenwand des Einschubteils kugelförmige Stützkörper, die lose angeordnet und somit in dem Zwischenraum beweglich sind. Das hat zur Folge, daß die Stützkörper im Zwischenraum verrutschen können und deshalb ihren Zweck, das Einschubteil zu versteifen, wenn im Zwischenraum ein Vakuum herrscht, nicht immer zuverlässig erfüllen. Somit kann der Benutzer sich nicht darauf verlassen, daß die Einführvorrichtung eine bestimmte gewünschte Form während einer bestimmten Handhabung eines Kolonoskops od.dgl. beibehält.

Schlauchförmige fiberoptische Instrumente haben, wie z.B. aus der US 4696544 bekannt ist, Anwendungsgebiete auch außerhalb der Medizin, beispielsweise zum Untersuchen von Rohrleitungen, Gefäßen und Maschinenteilen. Entsprechend ist auch der Begriff Untersuchungsobjekt im Rahmen der vorliegenden Erfindung zu verstehen.

Zum Einführen von Kolonoskopen sind ferner Einführvorrichtungen in Gebrauch, die ein einstückiges Gleitrohr von einer im Vergleich zum zugehörigen Kolonoskop hohen Biegesteifigkeit aufweisen, die nur verhältnismäßig geringfügige elastische Verbiegungen zuläßt. Der Innendurchmesser eines solchen Gleitrohrs für ein Kolonoskop mit 15 mm Außendurchmesser beträgt beispielsweise knapp 16 mm, so daß sich das Kolonoskop jederzeit leicht hindurchschieben läßt. Der Außendurchmesser des Gleitrohrs beträgt beispielsweise 19 mm. Der Einschubteil ist 40 cm lang; die Länge des zugehörigen Kolonoskops liegt üblicherweise zwischen 130 und 180 cm. Ein distaler Endbereich von etwa 10 cm Länge des Kolonoskops läßt sich mittels am proximalen Ende gelagerter Stellräder üblicherweise in vier Richtungen (oben/unten und links/rechts) bewegen.

Das Kolonoskop wird vom untersuchenden oder behandelnden Arzt vom Anus aus in das Kolon eingeführt. Damit eine vollständige Untersuchung des Kolons möglich ist, muß das Kolonoskop bis zum Coecum vorgeschoben werden. Dabei läßt sich die Bewegungsrichtung des Kolonoskops mit dessen beweglichem distalen Endbereich bestimmen. Dennoch ergeben sich an den Biegungen des Kolons, nämlich am Sigmoid und besonders an den beiden Kolonflexuren, regelmäßig Probleme mit Verletzungsgefahren, Schmerzen für den Patienten und krampfartigen Kontraktionen des Kolons bis hin zur Unmöglichkeit, die Untersuchung forzusetzen. Diese Probleme hängen damit zusammen, daß das Kolon weich ist und nur relativ wenig im Bauchraum befestigt ist. Die Hauptrichtung der Kraft, mit der das Kolonoskop vorgeschoben wird, verläuft nach einer Umlenkung nicht mehr in Richtung zum distalen Ende des Kolonoskops, sondern auf die leicht nachgebende Wand des Kolons, was für den Patienten unangenehm ist. In etwa 10 bis 15 % aller Fälle kann deshalb des Coecum nicht erreicht werden.

Diese Schwierigkeiten lassen sich mit den gebräuchlichen Einführvorrichtungen nur teilweise überwinden, da sie das Kolonoskop nur durch das Rektum und allenfalls noch das Sigmoid und das Kolon descendens zu führen vermögen. Spätestens an der linken Kolonflexur bleiben aber die beschriebenen Probleme bestehen. Außerdem wird es von vielen Patienten schon als unangenehm empfunden, daß dem Sigmoid von dem Gleitrohr ein nahezu geradliniger Verlauf aufgezwungen wird, da die Form des Gleitrohrs nicht willkürlich veränderbar ist. Wegen der hohen Steifigkeit des Gleitrohrs besteht bei unachtsamer Handhabung die Gefahr, daß das Kolon perforiert wird.

Aus der DE 3605169 A1 ist ein mechanisch-pneumatisches Manipulationssystem für Kolonoskope bekannt, bei dem mit einer Einführvorrichtung das Einführen eines Kolonoskops für Arzt und Patient erleichtert werden soll. Auch zu dieser Einführvorrichtung gehört ein flexibles Gleitrohr, in das sich das Kolonoskop einschieben läßt. Auf dem Gleitrohr ist ein Ballon oder eine Ballongruppe aus hochflexiblem Material angeordnet. Der Ballon oder die Ballongruppe liegt in entlüftetem Zustand fest an dem Gleitrohr an; in aufgeblasenem Zustand soll sich der Ballon oder die Ballongruppe in der Umgebung, also an der Darminnenwand, abstützen und dabei soll der Schaft des Kolonoskops mit Hilfe einer Mechanik vor- oder zurückbewegt werden können. Eine zweite Ballongruppe, die auf dem Kolonoskopschaft fest montiert ist, wird entlüftet, wenn das Kolonoskop gegenüber dem in der Umgebung abgestützten Gleitrohr verschoben werden soll, und wird aufgeblasen, wenn das Gleitrohr zusammen mit dessen Ballon oder Ballongruppe verschoben werden soll. Das Gleitrohr weist Längskanäle auf, von denen einer Druck- und Vakuumleitungen und ein anderer ein mechanisches Zug- und Schubsystem aufnehmen soll.

Diese bekannte Einführvorrichtung mit einem außen angeordneten aufblasbaren Ballon oder einer Gruppe solcher Ballons kann das Einführen eines Kolonoskops in einen menschlichen Darm nicht wesentlich erleichtern, da der Darm auf jede Dehnung, unabhängig davon, ob sie von einem ungeschützten Kolonoskop oder von einem oder mehreren aufgeblasenen Ballons hervorgerufen wird, in gleicher Weise mit spastischen, schmerzhaften Kontraktionen reagiert. Solche Kontraktionen treten auch distal des Kolonoskops auf und erschweren dessen weitere Vorwärtsbewegung. Für nicht medizinische Untersuchungsobjekte, wie beispielsweise Maschinenteile, die scharfkantige Innenkonturen haben können, eignet sich die bekannte Einführvorrichtung wegen der Verletzungsempfindlichkeit einer Ballonanordnung auf dem flexiblen Gleitrohr nicht.

Entsprechendes gilt auch für eine aus der DE 2823025 C2 bekannte Vorrichtung zum Transport eines Kolonoskops, bei dem das distale Ende des Kolonoskops durch einen dünnen, umgestülpten Schlauch mit dem distalen Ende eines konusförmigen Rohrs verbunden ist, das in den menschlichen Analring einführbar ist. Auf das proximale Ende des Rohrs ist ein Gehäuse aufschraubbar, welches das Kolonoskop umschließt und einen Anschlußstutzen aufweist. Wird durch den Anschlußstutzen ein Druckmedium eingeleitet, dann wird ein zwischen den beiden Schlauchenden liegender Abschnitt des Schlauchs in den Dickdarm hineingeschoben, wobei er sich ausstülpt und das Kolonoskop mit sich zieht. Dieses soll also durch Zug und nicht durch Schub in den Darm hineinbewegt werden. Beim Vordringen des Schlauchs soll dessen innerer Teil sich fortlaufend umstülpen, so daß er dann einen äußeren Schlauchteil bildet, der sich gegenüber der Darmschleimhaut nicht bewegt. Auch dadurch können jedoch schmerzhafte spastische Darmkontraktionen hervorgerufen werden. Selbst wenn es mit dieser bekannten Vorrichtung gelingt, ein Kolonoskop genügend weit, insbesondere bis zum Coecum vorzuschieben, ist es jedenfalls nicht möglich, den Schlauch im Darm zurückzulassen und ihn als Hilfe für ein erneutes Einführen des Kolonoskops zu verwenden, wenn dieses, beispielsweise zum Entfernen eines größeren Polypen, vorübergehend herausgezogen worden ist. Für technische Anwendungen eignet sich der umstülpbare und deshalb notwendigerweise dünne Schlauch nicht, da er an scharfen Kanten und Vorsprüngen des Untersuchungsobjekts leicht zerreißen kann.

Der Erfindung liegt die Aufgabe zugrunde, eine Einführvorrichtung für schlauchförmige fiberoptische Instrumente, insbes. Kolonoskope, Gastroskope und dgl. derart zu gestalten, daß sie das vollständige und nötigenfalls mehrfache Einführen eines solchen Instruments bis zum Ende des Untersuchungsobjekts wesentlich erleichtert und bei medizinischen Untersuchungen für den Patienten erträglicher macht und für den Arzt vereinfacht.

Die Aufgabe ist erfindungsgemäß ausgehend von einer Einführvorrichtung der eingangs beschriebenen Gattung dadurch gelöst, daß ein Teil der Stützkörper an der Außenseite der Innenwand fest angeordnet und der Rest der Stützkörper an der Innenseite der Außenwand fest angeordnet ist.

Die Stützkörper können Warzen, Wendeln, Rippen od.dgl. sein. Die feste Anordnung der Stützkörper kann sich durch einstückige Herstellung der Stützkörper mit der zugehörigen Innen- bzw. Außenwand oder durch Ankleben, Anschmelzen oder Anvulkanisieren gesondert hergestellter Stützkörper ergeben. Innen- und Außenwand bilden zusammen ein Einschubteil, das sich in beliebiger, z.B. an das Sigmoid und sogar an die beiden Kolonflexuren angepaßter Biegung versteifen läßt, indem man den Zwischenraum soweit evakuiert, daß die Stützkörper der Innenwand an denen der Außenwand anliegen. Durch das so verformte und fixierte Einschubteil läßt sich ein fiberoptisches Instrument, insbes. Kolonoskop, verhältnismäßig leicht einführen. Wird dann in den Zwischenraum wieder ein Fluid eingeleitet, so gewinnt der Einschubteil seine ursprüngliche Flexibilität zurück.

Die Innenwand ist vorzugsweise durch in den Zwischenraum eingeleitetes Fluid radial nach innen verformbar und dadurch an eine Mantelfläche eines durch die Einführvorrichtung hindurchgeführten fiberoptischen Instruments, insbes. Kolonoskops, anlegbar. Durch Einleiten eines Fluids unter Druck, beispielsweise Druckluft, in den Zwischenraum kann also die Innenwand des Einschubteils fest an ein durch die Einführvorrichtung hindurchgeführtes Instrument, insbes. Kolonoskop, angepreßt werden, ohne daß die Außenwand aufgebläht wird. In diesem Zustand ist die Einführvorrichtung noch flexibel und läßt sich wegen ihrer vorübergehend festen Verbindung mit dem fiberoptischen Instrument zusammen mit diesem vorschieben. So kann durch abwechselndes Nachschieben des Instruments allein und des Instruments mit Einführvorrichtung das Ende des Untersuchungsbereichs, beispielsweise das Coecum, problemlos erreicht werden.

Eine besonders feste gegenseitige Verzahnung und somit hohe Biegesteifigkeit des evakuierten Einschubteils ergibt sich, wenn die Stützkörper, die an der Außenseite der Innenwand angeordnet sind, in je eine Lücke zwischen Stützkörpern an der Innenseite der Außenwand eingreifen.

Der Zwischenraum kann in mehrere Kammern unterteilt sein, die einzeln oder gemeinsam evakuierbar sind und bei Bedarf unter Druck gesetzt werden können. Solche Kammern können beispielsweise durch Schweißnähte oder andere Nähte voneinander getrennt sein, die in der Art von Steppnähten die Innenwand mit der Außenwand verbinden. Die Kammern können sich geradlinig oder wendelförmig längs des Einschubteils erstrecken. Innen- und Außenwand können zwischen den Kammern einheitliche Wandbereiche bilden.

Bei einer bevorzugten Ausführungsform der Erfindung sind die an der Innenwand des Einschubteils fest angeordneten Stützkörper hülsenförmig gestaltet und greifen in je zwei benachbarte hülsenförmige Stützkörper ein, die an der Außenwand des Einschubteils fest angeordnet sind. Bei dieser Ausführungsform läßt sich das Einschubteil durch Evakuieren des Zwischenraums zwischen seiner Innenwand und seiner Außenwand besonders wirksam versteifen.

Die vorstehend beschriebene Ausführungsform kann dadurch weitergebildet sein, daß die an der Innenwand angeordneten Stützkörper durch Schlitze auf einem Teil ihrer axialen Länge radial aufweitbar und zusammendrückbar gestaltet sind. Damit wird erreicht, daß die an der Innenwand angeordneten, geschlitzten Stützkörper sich beim Evakuieren gut an die Stützkörper der Außenwand anschmiegen. Solche Schlitze ermöglichen es auch, das Einschubteil um besonders enge Radien zu biegen.

Dagegen ist es vorteilhaft, wenn die an der Außenwand angeordneten Stützkörper an ihren beiden Enden je einen nicht dehnbaren Ringwulst aufweisen, der die Schlitze der beiden benachbarten Stützkörper nur auf einem Teil ihrer axialen Länge übergreift, so daß sämtliche Teile des Zwischenraums in jeder Stellung des Einschubteils durch wenigstens einen Teil der Schlitze miteinander verbunden sind. Solche Ringwulste haben außerdem den Vorteil, daß sie jeglicher Tendenz der Außenwand zum Aufblähen entgegenwirken, wenn im Zwischenraum zwischen Innen- und Außenwand ein Überdruck herrscht.

Die Versteifung des Einschubteils bei evakuiertem Zwischenraum kann noch dadurch unterstützt werden, daß die an der Innenwand angeordneten Stützkörper außen eine Riffelung aufweisen, und daß die an der Außenwand angeordneten Stützkörper innen in entsprechender Weise geriffelt sind. Die somit einander radial gegenüberliegenden Riffelungen greifen bei evakuiertem Zwischenraum ineinander, so daß das Einschubteil in der Lage, die es unmittelbar vor dem Evakuieren des Zwischenraums eingenommen hat, formschlüssig festgelegt wird.

Ausführungsbeispiele der Erfindung werden im folgenden mit weiteren Einzelheiten anhand schematischer Zeichnungen erläutert. Es zeigen:
- Fig. 1: eine Gesamtansicht einer erfindungsgemäßen Einführvorrichtung mit Einschubteil und zugehörigen Apparaturen;
- Fig. 2: den Querschnitt II-II des Einschubteils in aufgeblähtem Zustand und in starker Vergrößerung;
- Fig. 3 a bis f: sechs aufeinanderfolgende Handhabungen eines Kolonoskops mit Einführvorrichtung;
- Fig. 4: eine zweite Ausführungsform eines erfindungsgemäßen Einschubteils im Querschnitt IV-IV in Fig. 5, in aufgeblähtem Zustand;
- Fig. 5: den noch stärker vergrößerten Längsschnitt V-V in Fig. 4; und
- Fig. 6: den Querschnitt VI-VI in Fig. 5.

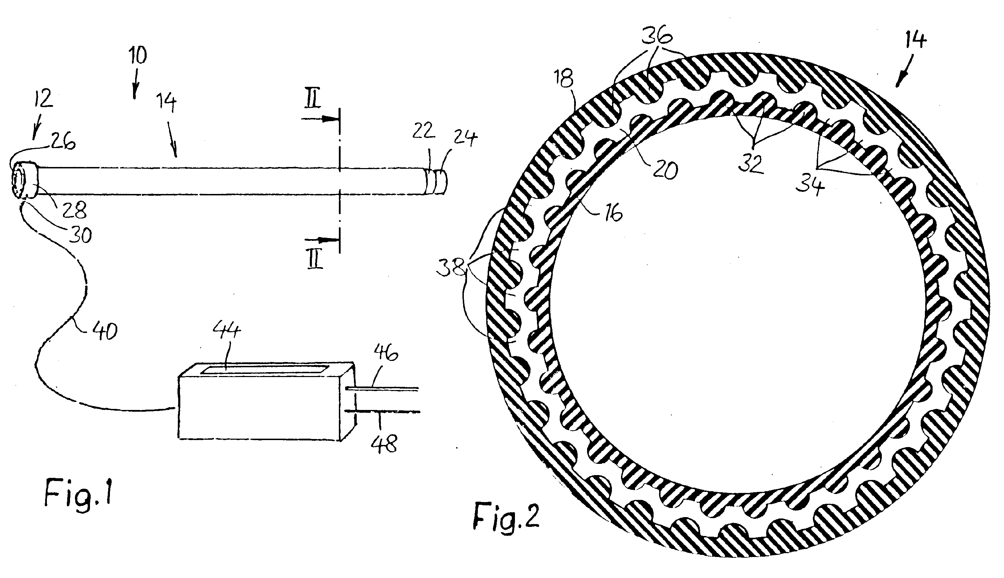

Hauptbestandteile der in Fig. 1 und 2 dargestellten Einführvorrichtung 10 sind ein Griffteil 12 und ein Einschubteil 14. Zum Einschubteil 14 gehören eine Innenwand 16, eine Außenwand 18, ein zwischen diesen gebildeter ringförmiger Zwischenraum 20, ein distaler Endbereich 22 und schließlich ein distaler Führungsring 24. Am Griffteil 12 und am distalen Endbereich 22 ist die Innenwand 16 dicht mit der Außenwand 18 verbunden, wodurch der Zwischenraum 20 abgeschlossen ist.

Der Griffteil 12 besteht gemäß Fig. 1 im wesentlichen aus einer Innenhülse 26, an der die Innenwand 16 dicht befestigt ist, und einer Außenhülse 28, an der die Außenwand 18 dicht befestigt ist. Innenwand 16 und Außenwand 18 können beispielsweise an der zugehörigen Hülse 26 bzw. 28 anvulkanisiert, angeklebt und/oder dicht festgeklemmt sein. Die beiden Hülsen 26 und 28 sind in ihrem proximalen Endbereich dicht miteinander verbunden, beispielsweise verschraubt.

An der Außenhülse 28 oder in einem Verbindungsbereich zwischen dieser und der Innenhülse 26 ist ein Anschluß 30 zum Einleiten und Absaugen eines Fluids in den bzw. aus dem Zwischenraum 20 angeordnet. An der Innenseite der Innenhülse 26 des Griffteils 12 sowie am distalen Endbereich 22 des Einschubteils 14 ist zweckmäßig je eine Gleitringdichtung angeordnet, die gegen das zugehörige fiberoptische Instrument abdichtet. In den Innenraum der Innenhülse 26 kann eine zusätzliche Saug- und/oder Spülleitung münden, damit durch die Einführvorrichtung Luft oder Sekret aus einem untersuchten Körperteil abgesaugt oder eine Spülflüssigkeit eingeleitet werden kann.

Innenwand 16 und Außenwand 18 sind von je einer schlauchförmigen Folie aus biegeweichem, körperverträglichem Kunststoff gebildet. Die Innenwand 16 hat bei der in Fig. 2 dargestellten Ausführungsform eine geringere Dicke als die Außenwand 18 und läßt sich deshalb leicht auch radial nach innen verformen, wenn im Zwischenraum 20 ein Überdruck erzeugt wird; die Außenwand 18 ist hingegen so fest und ggf. mit Fasern verstärkt, daß sie sich von einem im Zwischenraum 20 herrschenden Druck, der die Innenwand 16 schon merklich verformt, nicht aufblähen läßt.

An der Außenseite der Innenwand 16 sind gemäß Fig. 2 Stützkörper 32 gleichmäßig verteilt angeordnet; zwischen ihnen sind Lücken 34 freigelassen. In entsprechender Weise sind an der Innenseite der Außenwand 18 Stützkörper 36 und Lücken 38 derart angeordnet, daß jeder Stützkörper 32 einer Lücke 38, und jeder Stützkörper 36 einer Lücke 34 radial gegenübersteht. Solange im Zwischenraum 20 Umgebungsdruck herrscht, berühren die Stützkörper 32 und 36 einander nicht oder höchstens lose. Dieser Zustand ist in Fig. 2 dargestellt. Wenn der Zwischenraum 20 jedoch mehr oder weniger stark evakuiert wird, greift jeder Stützkörper 32 in eine Lücke 38, und jeder Stützkörper 36 greift in eine Lücke 34. Dadurch wird das Einschubteil 14 in einer beliebigen Form, die es vor dem Evakuieren eingenommen hatte, versteift.

Zur Benutzung der erfindungsgemäßen Einführvorrichtung wird der Anschluß 30 über einen Schlauch 40 mit einem Steuergerät 42 üblicher Art verbunden, das beispielsweise durch Betätigen eines wippenartigen Schalters 44 den Schlauch 40 wahlweise mit einer Druckluftleitung 46 oder einer Vakuumleitung 48 verbindet. Ein handelsübliches Kolonoskop 50 wird mit seinem distalen Ende 52 voran in das proximale Ende des Führungsrohrs 10 eingeführt und durch dessen Griffteil 12 sowie Einschubteil 14 hindurchgeschoben.

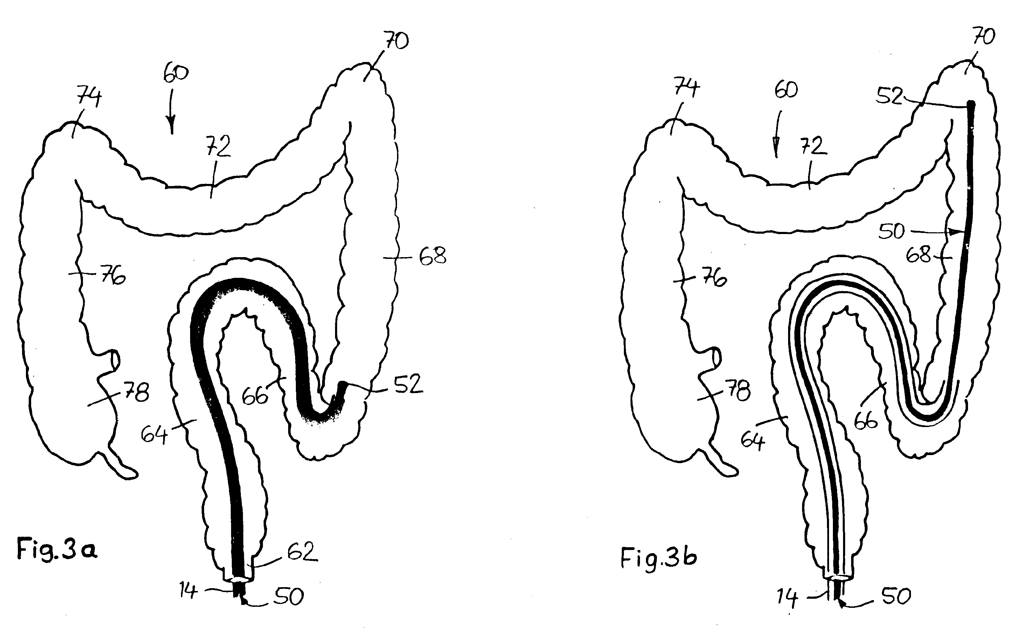

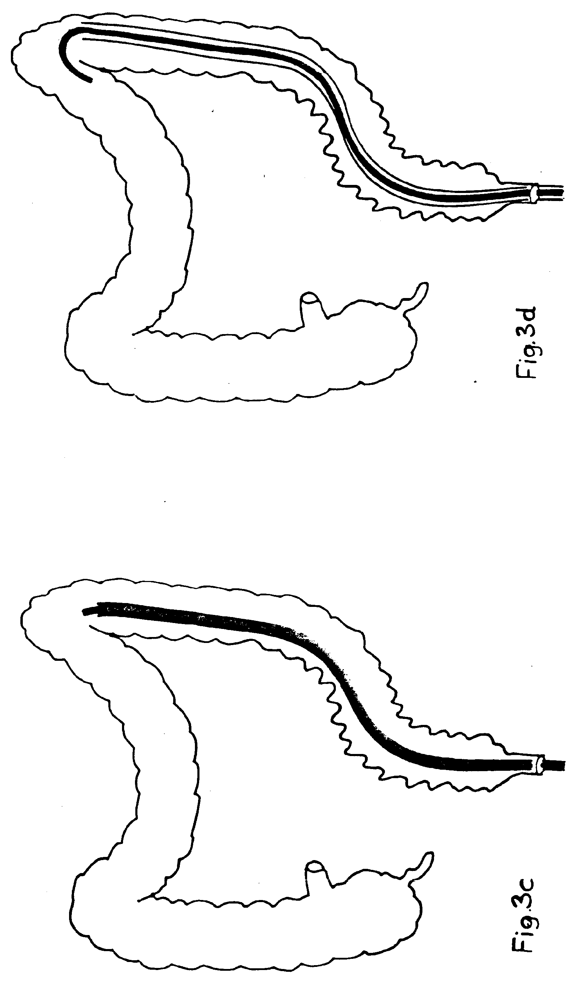

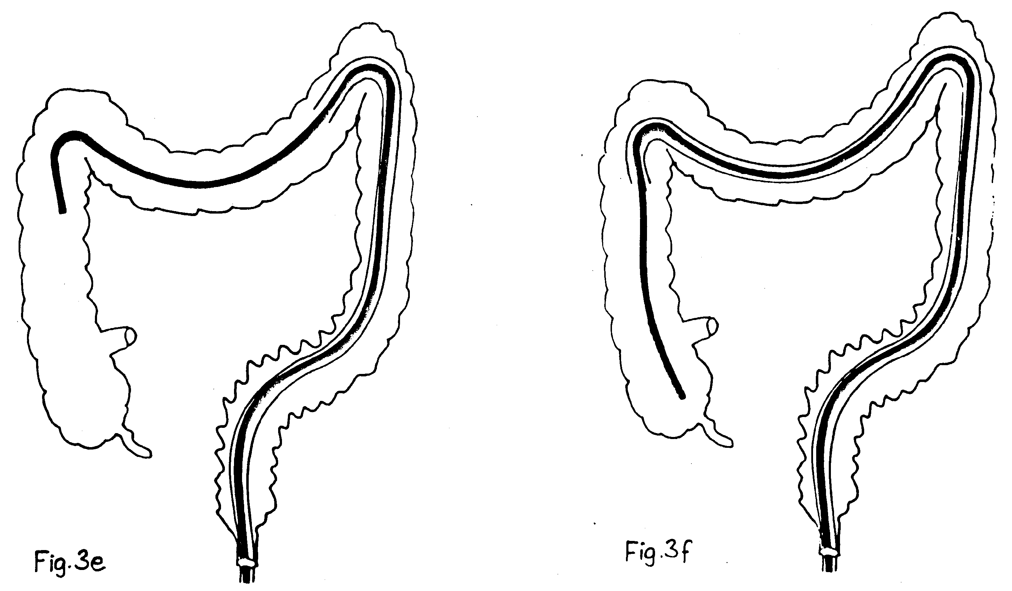

Die weitere Handhabung der Einführvorrichtung 10 und des Kolonoskops 50 findet am Patienten statt und ist in Fig. 3a bis 3f dargestellt. Dabei bedeutet jeweils die gemeinsame Darstellung des Einschubteils 14 und des Kolonoskops 50 als dicke Linie, daß in den Zwischenraum 20 Luft (oder ein anderes Fluid) mit Überdruck eingeleitet und dadurch die Innenwand 16 fest an das Kolonoskop 50 angelegt worden ist; das Einschubteil 14 ist dann genauso biegsam wie das Kolonoskop 50. Die Darstellung der Einschubteils 14 als dünnes, vom Kolonoskop 50 unterscheidbares Linienpaar bedeutet hingegen, daß im Einschubteil 14 ein Unterdruck herrscht, bei dem die Innenwand 16 das Kolonoskop 50 für Relativverschiebungen freigibt und fest an der Außenwand 18 anliegt, so daß der Einschubteil 14 versteift ist.

Gemäß Fig. 3a wird das Kolonoskop 50 mit dem an ihm fest anliegenden, biegeweichen Einschubteil 14 in das Kolon 60 eingeschoben, zunachst vom Anus 62 durch das Rektum 64 hindurch bis zum Übergang vom Sigmoid 66 zum Kolon descendens 68. In dieser Stellung wird das Einschubteil 14 durch Evakuieren seines Zwischenraums 20 versteift.

Gemäß Fig. 3b wird das Endoskop 50 durch die Einführvorrichtung 10 mit versteiftem Einschubteil 14 hindurch bis zur linken Kolonflexur 70 weitergeschoben.

Gemäß Fig. 3c wird die Versteifung des Einschubteils 14 durch Einleiten von Druckluft in seinen Zwischenraum 20 wieder aufgehoben; das Sigmoid 66 wird etwas geradegerichtet, und das Einschubteil 14 wird bis zur linken Kolonflexur 70 nachgeschoben.

In der Stellung gemäß Fig. 3d ist das Einschubteil 14 wieder versteift und das Kolonoskop 50 wird durch die linke Kolonflexur 70 hindurchgeschoben.

Gemäß Fig. 3e ist die Versteifung des Einschubteils 14 wieder aufgehoben worden; das Einschubteil 14 wird nun über die linke Kolonflexur 70 hinaus nachgeschoben und dann wieder versteift; anschließend wird das Endoskop 50 bis zur rechten Kolonflexur 74 und über diese hinaus vorgeschoben.

Gemäß Fig. 3f wird die Versteifung des Einschubteils 14 wieder aufgelöst; das Einschubteil 14 wird über die rechte Kolonflexur 74 hinaus nachgeschoben und anschließend wieder versteift, so daß das Kolonoskop 50 nun bis in das Coecum 78 weitergeschoben werden kann.

In jeder der beschriebenen Stellungen und in allen Zwischenstellungen der Einführvorrichtung 10 kann das Kolonoskop 50 vollständig herausgezogen werden, um beispielsweise einen mit einer Schlinge abgetragenen Tumor, der nicht durch das Kolonoskop 50 hindurch entfernt werden kann, nach außen zu transportieren. Anschließend kann das Kolonoskop 50 durch die in versteiftem Zustand im Kolon 60 gebliebene Einführvorrichtung 10 hindurch wieder eingeführt werden, und nötigenfalls können weitere Tumoren abgetragen und nach außen transportiert werden.

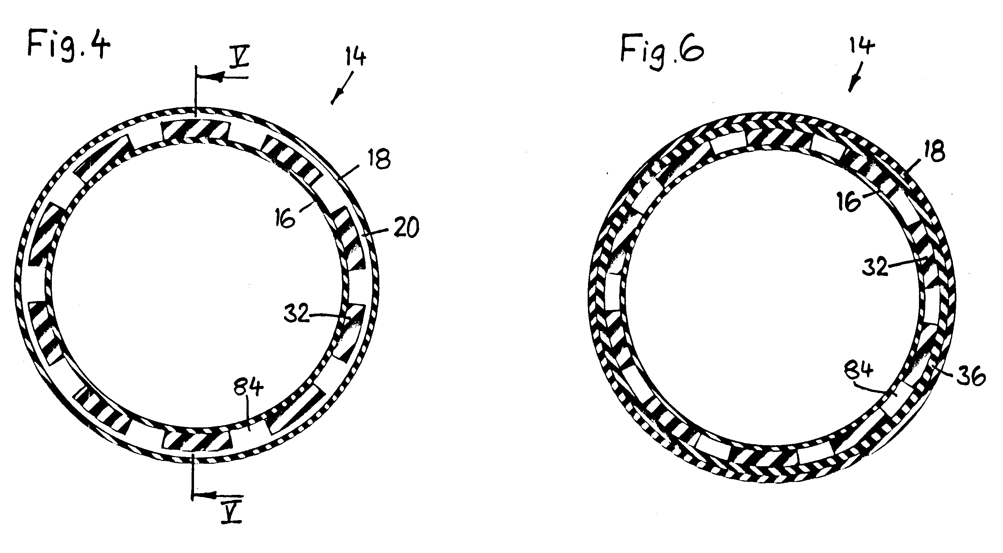

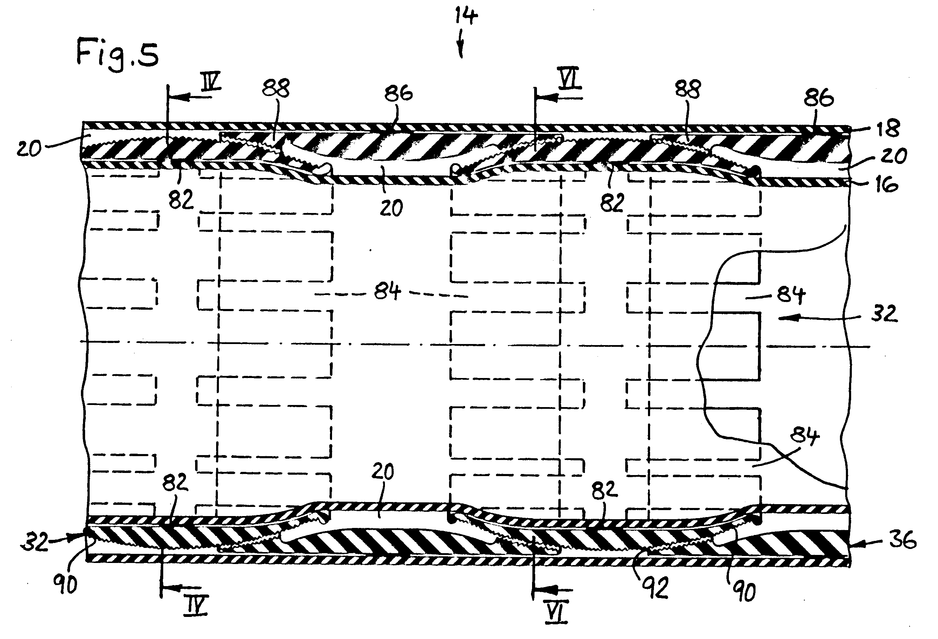

Bei der in Fig. 4 bis 6 dargestellten bevorzugten Ausführungsform der Erfindung sind an der Außenseite der Innenwand 16 ring- oder hülsenförmige Stützkörper 32 angeordnet, und mit je einer mittig an ihnen angeordneten ringförmigen Schweißung oder Klebung 82 an der Innenwand 16 befestigt. Jeder der Stützkörper 32 hat von seinen beiden Stirnseiten ausgehende achsparallele Schlitze 84, von denen jeder sich über nahezu die halbe axiale Breite des Stützkörpers 32 erstreckt. Die Schlitze 84 sind so breit, daß die ringförmigen Stützkörper 32 sich in ihren Endbereichen stark radial zusammendrücken und auch aufweiten lassen.

An der Innenseite der Außenwand 18 sind ring- oder hülsenförmige Stützkörper 36 angeordnet und ebenfalls mit je einer mittig an ihnen angeordneten ringförmigen Schweißung oder Klebung 86 an der Außenwand 18 befestigt. Jeder der Stützkörper 32 greift axial in zwei benachbarte Stützkörper 36 ein; in entsprechender Weise übergreift jeder der Stützkörper 36 die beiden benachbarten Stützkörper 32 auf einem Teil, z.B. etwa einem Drittel ihrer axialen Lange. Die Stützkörper 36 sind nicht geschlitzt, sondern an ihren beiden Enden mit je einem Ringwulst 88 versehen, der nicht oder nur geringfügig dehnbar ist.

Die Stützkörper 32 haben je eine sphärische Außenseite mit einer Riffelung 90; die Stützkörper 36 sind an der Innenseite ihrer Ringwulste 88 in komplementärer Weise spärisch ausgebildet und mit einer entsprechenden Riffelung 92 versehen. Wenn der wiederum zwischen Innenwand 16 und Außenwand 18 gebildete Zwischenraum 20 evakuiert wird, greifen die Riffelungen 90 und 92 einander überlappender Stützkörper 32 und 36 ineinander und fixieren die Stützkörper in der Stellung, die sie zu Beginn des Evakuierens eingenommen haben. Die Schlitze 84 bilden Längskanäle, die dafür sorgen, daß der Zwischenraum 20 zwischen der Innenwand 16 und der Außenwand 18 auf der gesamten Länge des Einschubteils 14 ohne Schwierigkeiten evakuiert werden kann. Durch die ineinandergreifenden Riffelungen 90 und 92 erhält das Einschubteil 14 in evakuiertem Zustand seines Zwischenraums 20 eine Steifigkeit, die um ein Vielfaches größer ist als die an sich sehr geringe Steifigkeit der Innenwand 16 und der Außenwand 18. Eine solche Versteifung läßt sich in beliebig gebogenem Zustand des Einschubteils 14 erzielen.

## Patentansprüche

1. Einführvorrichtung (10) für schlauchförmige fiberoptische Instrumente, insbes. Kolonoskope (50), Gastroskope und dgl. mit einem in ein Untersuchungsobjekt, insbes. menschliches Kolon (60), einschiebbaren, biegsamen Einschubteil (14), der zwischen einer Innenwand (16) und einer Außenwand (18) mindestens einen nach außen abgedichteten Zwischenraum (20) aufweist, in den sich ein Fluid einleiten läßt, wobei
- die Außenwand (18) des Einschubteils (14) von einem flexiblen, jedoch vom Fluid im Zwischenraum (20) nicht ballonartig aufblähbaren Schlauch gebildet ist,
- die Innenwand (16) des Einschubteils (14) ebenfalls von einem Schlauch gebildet ist, und
- der Zwischenraum (20) Stützkörper (32, 36) enthält, über die sich die Innenwand (16) und die Außenwand, (18) aneinander abstützen, wenn der Druck im Zwischenraum (20) einen vorbestimmten Betrag unterschreitet,
dadurch **gekennzeichnet**, daß ein Teil der Stützkörper (32) an der Außenseite der Innenwand (16) fest angeordnet ist und der Rest der Stützkörper (36) an der Innenseite der Außenwand (18) fest angeordnet ist.

2. Einführvorrichtung nach Anspruch 1,
dadurch **gekennzeichnet**, daß die Stützkörper (32), die an der Außenseite der Innenwand (16) angeordnet sind, in je eine Lücke (38) zwischen Stützkörpern (36) an der Innenseite der Außenwand (18) eingreifen.

3. Einführvorrichtung nach Anspruch 1 oder 2,
dadurch **gekennzeichnet**, daß die Stützkörper (32, 36) sich als umlaufende Versteifungen in Umfangsrichtung der Innenwand (16) bzw. Außenwand (18) erstrecken.

4. Einführvorrichtung nach Anspruch 3,
dadurch **gekennzeichnet**, daß die an der Innenwand (16) des Einschubteils (14) fest angeordneten Stützkörper (32) hülsenförmig gestaltet sind und in je zwei benachbarte hülsenförmige Stützkörper (36) eingreifen, die an der Außenwand (18) des Einschubteils (14) fest angeordnet sind.

5. Einführvorrichtung nach Anspruch 4,
dadurch **gekennzeichnet**, daß die an der Innenwand (16) angeordneten Stützkörper (32) durch Schlitze (84) auf einem Teil ihrer axialen Länge radial aufweitbar und zusammendrückbar gestaltet sind.

6. Einführvorrichtung nach Anspruch 5,
dadurch **gekennzeichnet**, daß die an der Außenwand (18) angeordneten Stützkörper (36) an ihren beiden Enden je einen nicht dehnbaren Ringwulst (88) aufweisen, der die Schlitze (84) der beiden benachbarten Stützkörper (36) nur auf einem Teil ihrer axialen Länge übergreift, so daß sämtliche Teile des Zwischenraums (20) in jeder Stellung des Einschubteils (14) durch wenigstens einen Teil der Schlitze (84) miteinander verbunden sind.

7. Einführvorrichtung nach einem der Ansprüche 1 bis 6,
dadurch **gekennzeichnet**, daß die an der Innenwand (16) angeordneten Stützkörper (32) außen eine Riffelung (90) aufweisen, und daß die an der Außenwand angeordneten Stützkörper (36) innen in entsprechender Weise geriffelt sind.

8. Einführvorrichtung nach einem der Ansprüche 1 bis 7,
dadurch **gekennzeichnet**, daß die Stützkörper (32, 36) mit der zugehörigen Innenwand (16) bzw. Außenwand (18) in einem Stück hergestellt sind.

9. Einführvorrichtung nach einem der Ansprüche 1 bis 8,
dadurch **gekennzeichnet**, daß die Innenwand (16) durch in den Zwischenraum (20) eingeleitetes Fluid radial nach innen verformbar und dadurch an einer Mantelfläche eines durch die Einführvorrichtung (10) hindurchgeführten fiberoptischen Instruments, insbes. Kolonoskops (50), anlegbar ist.

## Claims

1. An inserting means (10) for tubular, fiberoptic instruments, especially colonoscopes (50), gastroscopes, and the like, comprising a flexible insertion part (14) adapted to be pushed into an object to be examined, especially the human colon (60), and including, between an inner wall (16) and an outer wall (18), at least one intermediate space (20) which is sealed outwardly and into which a fluid can be introduced, in which
- the outer wall (18) of the insertion part (14) is formed by a flexible hose which, however, is not inflatable like a balloon by the fluid in the intermediate space (20),
- the inner wall (16) of the insertion part (14) likewise is formed by a hose, and
- the intermediate space (20) contains support elements (32, 36) through which the inner wall (16) and the outer wall (18) are mutually supported when the pressure in the intermediate space (20) fails to reach a predetermined value,
**characterized** in that several of the support elements (32) are firmly arranged at the outside of the inner wall (16) and the remainder of the support elements (36) are firmly arranged at the inside of the outer wall (18).

2. The inserting means as claimed in claim 1,
**characterized** in that the support elements (32) which are arranged at the outside of the inner wall (16) each engage in a gap (38) between support elements (36) at the inside of the outer wall (18).

3. The inserting means as claimed in claim 1 or claim 2,
**characterized** in that the support elements (32, 36) extend as circumferential reinforcements in circumferential direction of the inner wall (16) and the outer wall (18), respectively.

4. The inserting means as claimed in claim 3,
**characterized** in that the support elements (32) which are firmly arranged at the inner wall (16) of the insertion part (14) are formed as sleeves, each engaging in two adjacent sleeve-like support elements (36) which are firmly arranged at the outer wall (18) of the insertion part (14).

5. The inserting means as claimed in claim 4,
**characterized** in that the support elements (32) arranged at the inner wall (16) are designed to be readily expansible and compressible by slots (84) over part of their axial extension.

6. The inserting means as claimed in claim 5,
**characterized** in that the support elements (36) arranged at the outer wall (18) comprise a non-extensible annular bead (88) each at both ends which covers only part of the axial extension of the slots (84) of the two adjacent support elements (36) so that , with the insertion part (14) in any position, all parts of the intermediate space (20) communicate with each other through at least part of the slots (84).

7. The inserting means as claimed in any one of claims 1 to 6,
**characterized** in that the support elements (32) arranged at the inner wall (16) are formed with corrugations (90) at the outside, and that the support elements (36) arranged at the outer wall are formed with corresponding corrugations at the inside.

8. The inserting means as claimed in any one of claims 1 to 7,
**characterized** in that the support elements (32, 36) are made in one piece with the respective inner (16) and outer walls (18).

9. The inserting means as claimed in any one of claims 1 to 8,
**characterized** in that the inner wall (16) is deformable radially inwardly by fluid introduced into the intermediate space (20), thereby being adapted to engage a sheath surface of a fiberoptic instrument, especially a colonoscope (50) passed through the inserting means (10).

## Revendications

1. Dispositif d'introduction (10) pour instruments tubulaires flexibles à fibres optiques, notamment colonoscopes (50), gastroscopes et similaires, comportant une pièce d'insertion (14) flexible, insérable dans un objet à examiner, notamment le côlon humain (60) et qui présente entre une paroi inteme (16) et une paroi externe (18) au moins un intervalle (20) étanche vis-à-vis de l'extérieur et dans lequel on peut envoyer un liquide,
- la paroi externe (18) de la pièce d'insertion (14) étant constituée par un tube flexible, mais qui ne peut être ballonné par le liquide se trouvant dans l'intervalle (20),
- la paroi externe (16) de la pièce d'insertion (14) étant également constituée par un tube souple et
- l'intervalle (20) contenant des corps de soutien (32, 36) par l'intermédiaire desquels la paroi inteme (16) et la paroi externe (18) s'appuient l'une à l'autre lorsque la pression dans l'intervalle (20) tombe en dessous d'une valeur prédéterminée,
caractérisé en ce qu'une partie des corps de soutien (32) est solidaire de la face externe de la paroi interne (16) et que le reste des corps de soutien (36) est solidaire de la face interne de la paroi exteme (18).

2. Dispositif d'introduction selon la revendication 1, caractérisé en ce que les corps de soutien (32) disposés sur la face externe de la paroi interne (16) s'engrènent dans des interstices (38) respectifs entre les corps de soutien (36) de la face interne de la paroi externe (18).

3. Dispositif d'introduction selon la revendication 1 ou 2, caractérisé en ce que les corps de soutien (32, 36) s'étendent sous forme de renforts circonférentiels dans la direction circonférentielle de la paroi interne (16) ou de la paroi externe (18).

4. Dispositif d'introduction selon la revendication 3, caractérisé en ce que les corps de soutien solidaires de la paroi interne (16) de la pièce d'insertion (14) sont conçus sous forme de manchons et s'engrènent entre deux corps de soutien (36) voisins en forme de manchons, solidaires de la paroi exteme (18) de la pièce d'insertion (14).

5. Dispositif d'introduction selon la revendication 4, caractérisé en ce que les corps de soutien (32) disposés sur la paroi interne (16) sont conçus de façon à être radialement expansibles et compressibles sur une partie de leur longueur axiale, grâce à des fentes (84).

6. Dispositif d'introduction selon la revendication 5, caractérisé en ce que les corps de soutien (36) disposés sur la paroi exteme (18) présentent à chacune de leurs extrémités un bourrelet annulaire (88) non extensible qui ne recouvre les fentes (84) des deux corps de soutien (36) voisins que sur une partie de leur longueur axiale de sorte que toutes les parties de l'intervalle (20) sont reliées par au moins une partie des fentes (84), quelle que soit la position de la pièce d'insertion (14).

7. Dispositif d'introduction selon une des revendications 1 à 6, caractérisé en ce que les corps de soutien (32) disposés sur la paroi inteme (16) présentent extérieurement un cannelage (90) et que les corps de soutien (36) disposés sur la paroi externe sont cannelés intérieurement de façon correspondante.

8. Dispositif d'introduction selon une des revendications 1 à 7, caractérisé en ce que les corps de soutien (32, 36) et la paroi interne (16) ou externe (18) correspondante sont fabriqués d'une seule pièce.

9. Dispositif d'introduction selon une des revendications 1 à 8, caractérisé en ce que la paroi inteme (16) est déformable radialement vers l'intérieur par un fluide envoyé dans l'intervalle (20) et peut ainsi s'accoller à la surface de la gaine d'un instrument à fibres optiques, notamment un colonoscope (50), guidé à travers le dispositif d'introduction (10).
